(19) Europäisches Patentamt
European Patent Office
Office européen des brevets

(11) EP 0 420 487 B1

(12) **EUROPEAN PATENT SPECIFICATION**

(45) Date of publication and mention of the grant of the patent:
**25.09.1996 Bulletin 1996/39**

(51) Int Cl.6: **C07D 487/08**, C07D 207/12, C07D 207/48 // (C07D487/08, 209:00, 209:00)

(21) Application number: **90310239.0**

(22) Date of filing: **19.09.1990**

(54) **Process for optically active 2-alkyl-2,5-diazabicyclo[2.2.1]heptanes**

Verfahren zur Herstellung von optisch aktiven 2-Alkyl-2,5-diazabicyclo[2.2.1]heptanen

Procédé de préparation de 2-alkyl-2,5-diazabicyclo[2.2.1]heptanes optiquement actif

(84) Designated Contracting States:
**AT BE CH DE DK ES FR GB GR IT LI LU NL SE**

(30) Priority: **25.09.1989 US 412072**

(43) Date of publication of application:
**03.04.1991 Bulletin 1991/14**

(73) Proprietor: **PFIZER INC.**
**New York, N.Y. 10017 (US)**

(72) Inventors:
- **Braish, Tamim Fehme**
**Ledyard, Connecticut, 06339 (US)**
- **Fox, Darrell Eugene**
**Pawcatuck, Connecticut, 06379 (US)**

(74) Representative: **Moore, James William, Dr.**
**Pfizer Limited**
**Ramsgate Road**
**Sandwich Kent CT13 9NJ (GB)**

(56) References cited:
- **JOURNAL OF ORGANIC CHEMISTRY, vol. 31, 1966, pages 1059-1062; P.S. PORTOGHESE et al.: "Bycyclic bases. Synthesis of 2,5-diazabicyclo[2.2.1]heptanes"**
- **AUSTRALIAN JOURNAL OF CHEMISTRY, vol. 20, 1967, pages 1493-1509; R.H. ANDREATTA et al.: "Synthesis of Cis and Trans isomers of 4-chloro-L-proline, 4-bromo-L-proline, and 4-amino-L-proline"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to the preparation of 1S,4S- and 1R,4R-2-alkyl-2,5-diazabicyclo[2.2.1]-heptane intermediates having utility in the preparation of antibacterial quinolones such as those disclosed in U.S. Patent No. 4,775,668.

A method for the synthesis of 2,5-diazabicyclo-[2.2.1]heptanes has been described by Portoghese et al., J. Org. Chem., 31, 1059 (1966). According to this method, hydroxy-L-proline is transformed into tritosylhydroxy-L-prolinol which is first reacted with benzylamine and then with hydrogen iodide, phosphorus, and acetic acid to form N-benzyl-2,5-diazabicyclo-[2.2.1]heptane dihydroiodide. U.S. Patent No. 3,947,445 follows a similar procedure and then converts the dihydroiodide through a three step procedure into 2-methyl-2,5-diazabicyclo[2.2.1]heptane.

In U.S. Patent No. 5,036,153, we describe another method for the preparation of said optically active 2,5-diaza-2-alkylbicyclo[2.2.1]heptanes of the formula (IX) below from trans-4-hydroxy prolines of the formula (I) below.

The over-all processes of the present invention for the preparation of optically active 2,5-diaza-2-alkylbicyclo[2.2.1] heptanes (IX) from 4-hydroxyproline (I) are shown in Schemes I and II. In these schemes, all of the compounds depicted are chiral and optically active. Viewed as formulas which depict absolute stereochemistry, they depict, for example, trans-4-hydroxy-L-proline, also known simply as hydroxyproline, of the formula (I), and (1S,4S)-2-alkyl 2,5-diaza[2.2.1] heptanes, of the formula (IX). Viewed as formulas which depict relative stereochemistry, they also depict the corresponding enantiomers, for example, trans-4-hydroxy-D-proline (I) and (1R,4R)-2-alkyl-2,5-diaza[2.2.1]heptanes (IX). In these formulas

R, $R^1$ and $R^3$ are each independently ($C_1$-$C_6$)alkyl;
$R^2$ and $R^4$ are each independently ($C_1$-$C_6$)alkyl, trifluoromethyl or

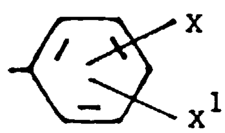 ;

and
X and $X^1$ are each independently hydrogen, ($C_1$-$C_6$)alkyl, bromo, chloro, trifluoromethyl, methoxy or nitro.

In particular, the present invention is directed to the process steps:

(VII)→(VIII) [carried out by the agency of at least one molar equivalent of an alkali metal carbonate salt in a reaction inert solvent];

Scheme I

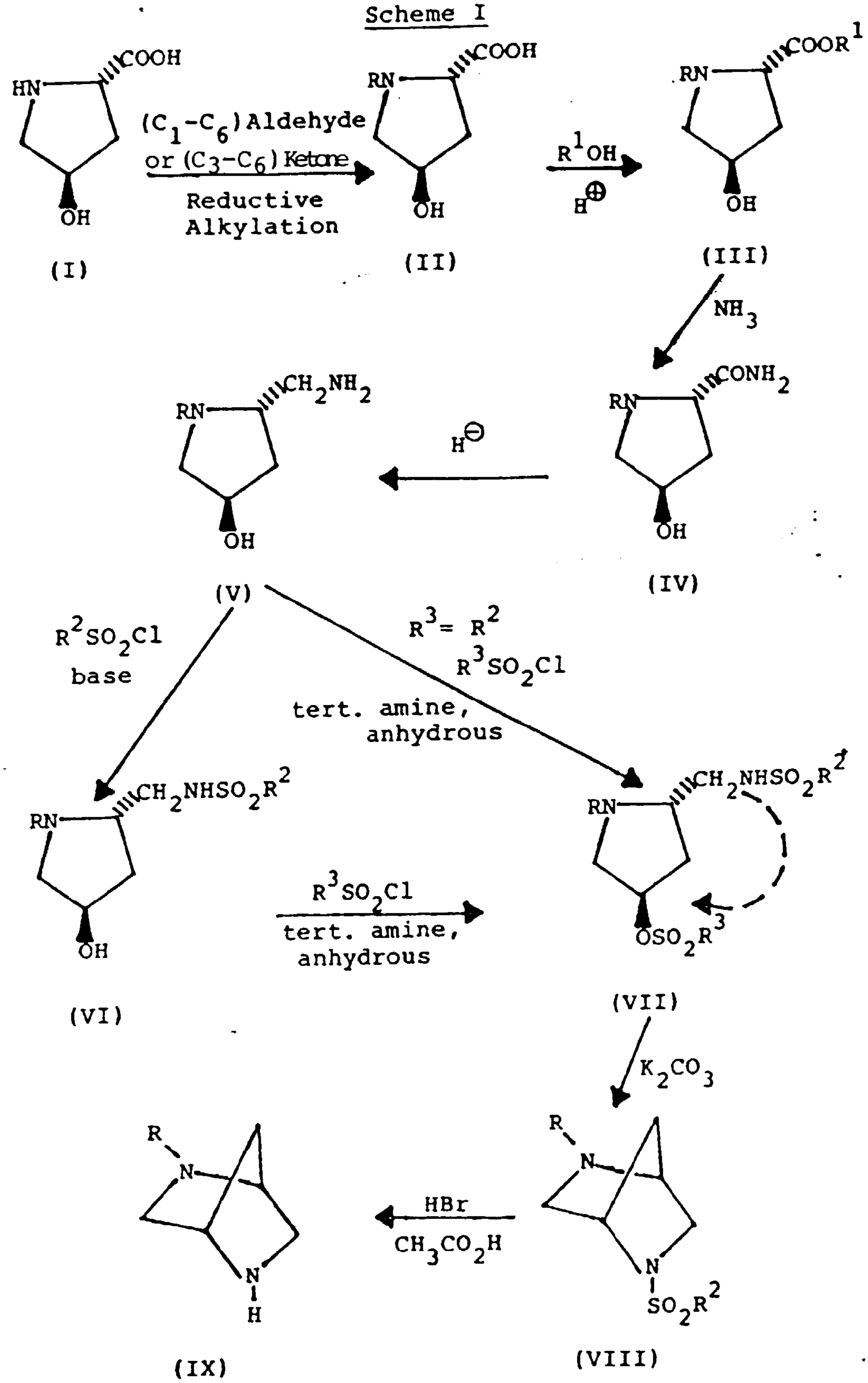
(I)
$(C_1-C_6)$ Aldehyde
or $(C_3-C_6)$ Ketone
Reductive
Alkylation
(II)
$R^1OH$
$H^{\oplus}$
(III)
$NH_3$
(IV)
$H^{\ominus}$
(V)
$R^2SO_2Cl$
base
$R^3 = R^2$
$R^3SO_2Cl$
tert. amine,
anhydrous
(VI)
$R^3SO_2Cl$
tert. amine,
anhydrous
(VII)
$K_2CO_3$
(VIII)
HBr
$CH_3CO_2H$
(IX)

Scheme II

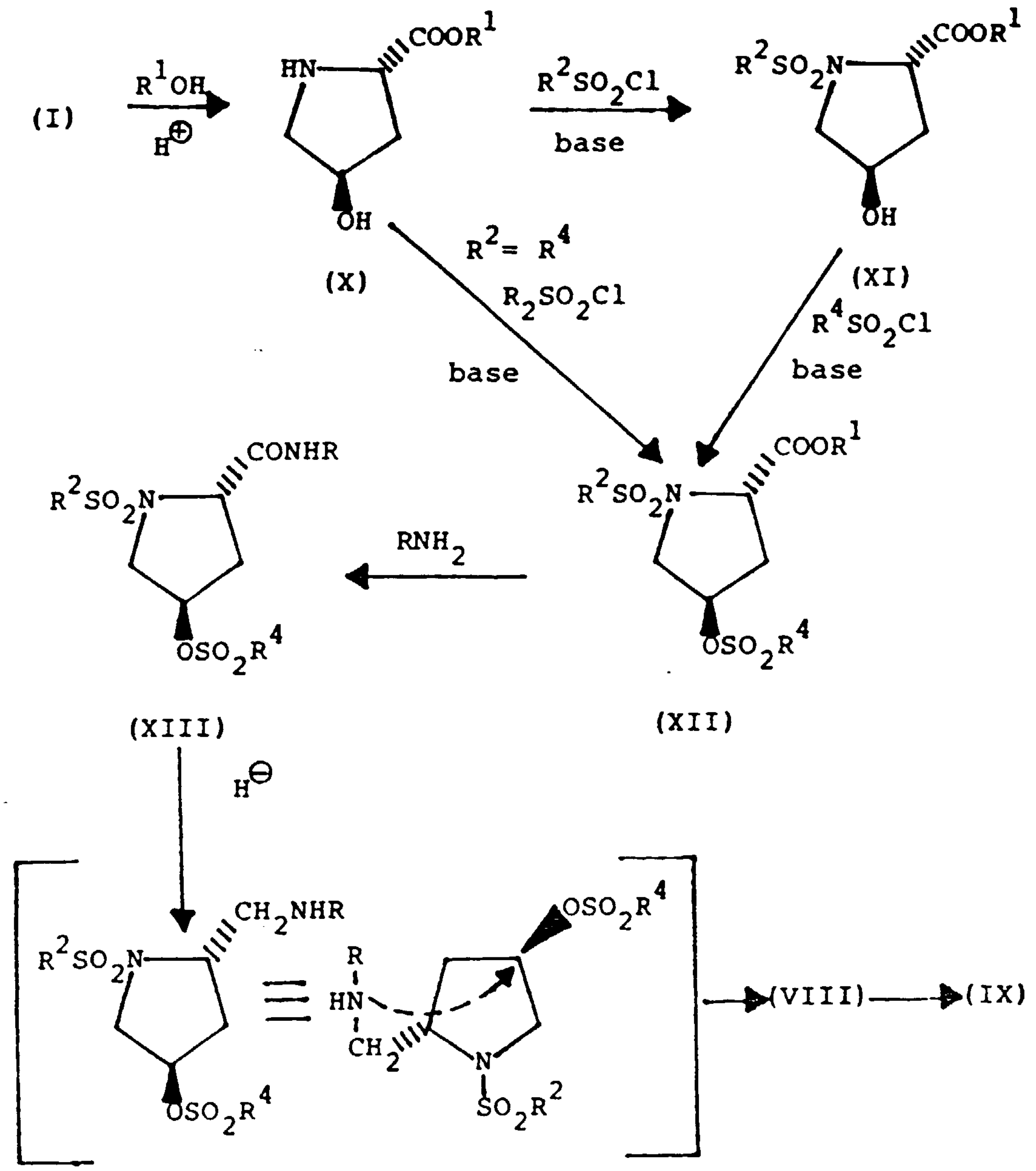


(XIV)

(VII)→(VIII)→(IX) ;

(I)→(II)→(III)→(IV)→(V)→(VII)→(VIII);

(XIII)→[(XIV)]→(VIII) [carried out by the agency of a hydride reducing agent in a reaction inert solvent, without isolation of the intermediate amine];

(XIII)→[(XIV)]→(VIII)→(IX); ); and

(I)→(X)→(XII)→(XIII)→[(XIV)]→(VIII).

The expression "reaction inert solvent" refers to a single or multicomponent solvent, the component(s) of which do not interact with starting materials, reagents, intermediates or products in a manner which adversely affects the yield of the desired product.

In the conversion of (VII) to (VIII), the : preferred reagent is $K_2CO_3$ and the preferred solvent is a lower alcohol, particularly methanol. In the conversion of (XIII) to (VIII), the preferred hydride reducing agent is $LiAlH_4$ and the preferred solvent is an ether, particularly diethylether or tetrahydrofuran.

In these processes, the preferred values of R and $R^3$ are each methyl; of $R^1$ is methyl or ethyl; and of each of $R^2$ and $R^4$ is methyl or 4-methylphenyl. Because the number of steps is reduced, it is preferred that $R^2$ and $R^3$ or $R^4$ have the same value.

The present invention is also directed to optically active intermediates of the relative or absolute stereochemical formula

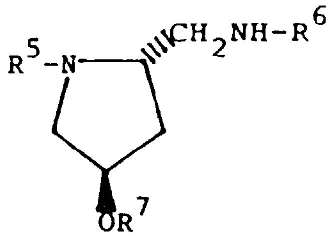

(XV)

wherein in a first alternative,

$R^5$ is $(C_1-C_6)$ alkyl;
$R^6$ is $SO_2R^2$;
$R^2$ is as defined above;
$R^7$ is hydrogen or $SO_2R^3$; and
$R^3$ is as defined above; or

in a second alternative,

$R^6$ is $(C_1-C_6)$alkyl;
$R^5$ is $SO_2R^2$;
$R^2$ is as defined above;
$R^7$ is $SO_2R^4$; and
$R^4$ is as defined above;

The preferred compounds of the formula (XV) are in the first alternative having $R^5$ as methyl, $R^2$ as methyl or 4-methylphenyl, and, when $R^7$ is $SO_2R^3$, $R^3$ as methyl;

and in the second alternative having $R^6$ as methyl, and $R^2$ and $R^4$ as methyl or 4-methylphenyl.

The various process steps of the present invention are readily carried out.

The initial step according to Scheme I involves conventional reductive alkylation of a trans-hydroxyproline (I) with a $(C_1-C_6)$aliphatic aldehyde or $(C_3-C_6)$aliphatic ketone appropriate to the desired N-alkyl substituent (e.g., formaldehyde → methyl, hexanal→hexyl, acetone→isopropyl). This reductive alkylation is carried out under typical hydrogenation conditions, best over a noble metal catalyst, preferably palladium. The catalyst can be a noble metal per se, or an oxide or salt, reduced to the active metal catalyst under the conditions of hydrogenation, or a noble metal catalyst on a support such as carbon or alumina. In the present instance the most preferred catalyst is Pd/C. The reaction inert solvent includes water, an organic solvent such as ethanol or a mixed solvent such as aqueous alcohol. When R is methyl, the preferred source of formaldehyde is simply aqueous formaldehyde and in this case water alone is the preferred solvent. At least one molar equivalent of the aldehyde or ketone is usually employed, and when this reagent (like formaldehyde) is readily available, it can be employed in large excess in order to reduce reaction time and maximize the stoichiometric yield from the generally more valuable hydroxyproline. Temperature is not critical, temperatures of 0-50°C being generally satisfactory, and ambient temperature, avoiding the cost of heating or cooling, is most preferred. Likewise, pressure is not critical, but low to moderate pressures (e.g., 1-8 atmospheres) are preferred in order to avoid the need for expensive, high pressure reactors.

The second step in Scheme I (II→III) involves conventional conversion of a carboxylic acid group to a $(C_1-C_6)$ carboxylate ester, preferably accomplished by simple strong acid catalyzed esterification with an alcohol, usually employed in gross excess and also as solvent. Suitable strong acids are such as HCl, $H_2SO_4$ and $R^2SO_3H$ where $R^2$ is defined as above, are generally employed in anhydrous or near anhydrous form in amounts ranging from truly catalytic (e.g., 10 mol %), but generally in excess of the 100 mol % necessary to neutralize the tertiary amine group. Temperature is not critical, 0-50°C being generally satisfactory and room temperature particularly convenient.

In the third step of Scheme I (III→IV), the lower alkyl ester group is conventionally converted to a carboxamide group by the action of ammonia in a reaction inert solvent. When water is the solvent, undiluted, concentrated ammonia is preferred. When the solvent is a lower alcohol such as methanol, anhydrous ammonia, saturated into the solvent,

is preferred. Temperature is not critical, 0-50°C being generally satisfactory, with ambient temperatures being especially convenient.

In the fourth step of Scheme I, the carbamoyl group in (IV) is reduced with a hydride reducing agent to the aminomethyl group in (V). Hydride reducing agents, such as diisobutyl aluminum hydride or lithium aluminum hydride which show a high level of activity in reducing amides to amines, are preferred reagents in this reduction. It is preferred to use an excess of these reagents, eg., as many as 6 mols of diisobutyl aluminum hydride or 1.5 mols of lithium aluminum hydride per mole of amide. The reduction is carried out in a reaction inert solvent, usually an ether such as diethylether or tetrahydrofuran. Temperature is not critical, 0-50°C generally being satisfactory and ambient temperatures most preferred.

In Scheme I, when $R^2$ and $R^3$ are different, the conversion of (V) to (VII) will require two steps. In the first of these, the sulfonamide is best selectively formed by sulfonylation of the amine with substantially one molar equivalent of the appropriate sulfonyl chloride in the presence of a sufficient amount of a base (such as n-butyllithium) to neutralize coproduced hydrogen chloride, in a reaction inert solvent, suitably an ether such as tetrahydrofuran. This reaction is generally carried out at a reduced temperature (e.g., -50 to +15°C), a temperature near the middle of this range (e.g., -10 to -15°C), being particularly satisfactory. In this case, subsequent O-sulfonylation (VI→VII) is readily accomplished using at least one molar equivalent of $R^2SO_2Cl$ in the presence of at least one molar equivalent of a tertiary amine such as triethylamine, generally in an aprotic solvent such as an ether (e.g., tetrahydrofuran). Temperature in this second stage is not critical, with a temperature in the range of 0-50°C generally satisfactory and ambient temperature usually preferred.

As noted above in the preferred routes $R^2$ and $R^3$ are the same, in which case concurrent N- and O- sulfonylation is readily accomplished under the latter conditions, except that at least two equivalents each of the sulfonylchloride ($R^3SO_2Cl$) and the tertiary amine are employed.

At the heart of the present invention is the cyclization of the bis-sulfonylated derivative (VII) to form the 2-alkyl-5-(alkane- or benzene-)sulfonyl derivative (VIII). This cyclization is readily accomplished by the agency of at least one molar equivalent of an alkali metal carbonate (e.g., $K_2CO_3$ is particularly well-suited) in a reaction inert solvent such as a lower alcohol (e.g., methanol is particularly well-suited).

In the first step of Scheme II, the hydroxyproline (I) is converted to the ester (X) best accomplished by the methods described above for the esterification of N-alkylhydroxyprolines (II→III). Bis-sulfonylation of (X) to form the compound of the formula (XII) and its ammonolysis to the amide (XIII) are also accomplished by the methods described above, except that the amine $RNH_2$ is substituted for $NH_3$.

However, in marked contrast to Scheme I, in Scheme II, the hydride reduction of the carbamoyl group to an alkylamino group and cyclization are now accomplished in a single step, viz., by the action of hydride reducing agent as described above, with the expected product, (XIV), spontaneously cyclizing to form the desired 2-alkyl-5-alkane- or benzenesulfonyl derivative (VIII).

Finally, in order to form the amine (IX), according to either Scheme, the N-sulfonyl group is removed by conventional reductive or hydrolytic methods. This is best accomplished with HBr in acetic acid as reagent, conveniently with isolation of (IX) in the form of its dihydrobromide salt. Again temperature is not critical, 0-50°C being fully satisfactory and ambient temperature generally preferred. Those skilled in the art will understand that sodium in liquid ammonia represents an alternative reductive method for removal of the sulfonyl group, while use of strong aqueous acid (HCl, $H_2SO_4$, etc.) represents an alternative hydrolytic method for its removal.

The starting materials required in the operation of the present invention are readily available. Thus, trans-hydroxy-L-proline is a natural aminoacid which is commercially available, while its enantiomer is available according to the method of Baker, et al., J. Org. Chem., 46, pp. 2954-2960 (1981).

The amines of the formula (IX) are used as the source of side chain in the preparation of certain antibacterial quinolones such as those disclosed in U.S. Patent 4,775,668, cited above.

The present invention is illustrated by the following Examples. Nomenclature used herein is based on Rigaudy and Klesney, IUPAC Nomenclature of Organic Chemistry, 1979 Ed., Pergammon Press, New York, 1979.

EXAMPLE 1

trans-N-Methyl-4-hydroxy-L-proline (II, $R=CH_3$)

To 40 g (305 mmol) of trans-4-hydroxy-L-proline in 80 ml of water was added 80 ml of 30% aqueous formaldehyde solution and 7.0 g of 5% palladium on carbon catalyst (50% wet), and the mixture was hydrogenated at 50 psig using a Parr Shaker. After 24 hours, the catalyst was recovered by filtration over diatomaceous earth and the filtrate evaporated under reduced pressure to provide 43.5 g (98.3%) of title product; mp 140-142°C (decomposition); $^1$H-NMR ($D_2O$): 4.65 (m, 1H), 4.20 (dd, 1H) , 3.97 (dd, 1H), 3.2 (dm, 1H), 2.50 (m, 1H), 2.25 (m, 1H); $[alpha]_D$ = -54.8° (c=1.18, $H_2O$)

EXAMPLE 2

trans-N-methyl-4-hydroxy-L-proline Methyl Ester (III, R=$R^1$=$CH_3$)

Title product of the preceding Example (100 g, 690 mmol) was suspended in 600 ml of methanol and anhydrous HCl gas was bubbled through the reaction mixture until it became homogeneous. The reaction was then heated to reflux for 16 hours, after which it was cooled and the solvent was replaced with 150 ml of water. 200 g (1.44 mol) of potassium carbonate was then added carefully at 0°C and the product was extracted with 4 x 200 ml portions of ethyl acetate. The combined organic layers were dried ($Na_2SO_4$) and evaporated to provide 87 g (72%) of product as a white solid; mp 53-54°C; $^1$H-NMR($D_2O$) 4.85 (s, 3H), 4.73 (m, 2H), 3.90 (s, 3H, N-methyl), 3.58 (m, 1H), 3.45 (m, 1H), 2.54 (m, 1H), 2.37 (m, 1H); $[alpha]_D$ = -80.0° (c=1.038, $CH_3OH$).

EXAMPLE 3

(2S,4R)-1-Methyl-2-carbamoyl-4-hydroxypyrrolidine (IV, R=$CH_3$)

Title product of the preceding Example (20 g, 126 mmol) was dissolved in 40 ml of ice cold, saturated $NH_4OH$, and the resulting solution then warmed to room temperature. After stirring for 24 hours the solvent was removed under high vacuum to produce a quantitive yield of present title product as a white, crystalline solid; mp 138-140°C; Anal. C 49.99, H 8.40, N 18.27; calcd. C 49.97, H 8.40, N 19.43; $^1$H-NMR ($D_2O$) 4.43 (m, 1H), 3.42 (dd, 1H), 3.25 (AB pattern, 1H), 2.36 (s, 3H), 2.33 (M, 1H), 2.1 (m, 2H); $[alpha]_D$ = -105.48° (c=0.953, $CH_3OH$).

EXAMPLE 4

(2S,4R)-1-Methyl-2-aminomethyl-4-hydroxypyrrolidine (V, R=$CH_3$)

Title product of the preceding Example (15 g, 104 mmol) was suspended in 75 ml of tetrahydrofuran and 572.5 ml (572.5 mmol) of diisobutyl aluminum hydride (1M solution in hexanes) was added over a period of 15 minutes. The mixture was then heated to reflux for two days and judged complete by monitoring with thin layer chromatography. Diatomaceous earth (30 g) was then added to the reaction and while cooling with an ice bath, 300 ml of methanol was added dropwise. The slurry was then filtered and the solvents were evaporated to provide 8.1 g of a colorless oily product (60%); $^{13}$C-NMR ($D_2O$) 69.5 (CH), 66.8 (CH), 64.9 ($CH_2$), 44.0 ($CH_2$), 41.0 ($CH_3$), 39.5 ($CH_2$); $[alpha]_D$ - 61°94° (c=0.956, $CH_3OH$).

EXAMPLE 5

(2S,4R)-1-Methyl-2-[(4-methylbenzenesulfonylamino)methyl]-4-hydroxypyrrolidine (VI, R=CH3, $R^2$= p-$CH_3C_6H_4$)

To title product of the preceding Example (7.3 g, 56.2 mmol) in 200 ml of tetrahydrofuran at -10°C was added 22.46 ml of n-butyllithium (56.2 mmol, 2.5 M in hexanes) over a period of 30 minutes. p-Toluenesulfonyl chloride (10.2 g, 53.3 mmol) in 10 ml of tetrahydrofuran was then added. After stirring the mixture for two hours at -10°C, 20 ml of water were added and the reaction was extracted with 2 x 140 ml of methylene chloride. The combined organic layers were dried ($Na_2SO_4$) and evaporated at reduced pressure to provide 15 g (94.3%) of present title product as a light yellow oil; $^{13}$C-NMR ($CDCl_3$) 143.4, 136.5, 129.7, 127.0, 69.1, 64.7, 62.4, 43.0, 40.0, 38.2, 21.5; $[alpha]_D$ = -34.67° (c=0.90, $CH_3OH$).

EXAMPLE 6

(2S,4R)-1-Methyl-2-[(4-methylbenzensulfonylamino)methyl]-4-(methanesulfonyloxy)pyrrolidine (VII, R=$R^3$=$CH_3$, $R^2$=p$CH_3C_6H_4$)

To title product of the preceding Example (1.0 g, 3.5 mmol) in 20 ml of tetrahydrofuran was added 0.49 ml (3.5 mmol) of triethylamine and 0.27 ml (3.5 mmol) of methanesulfonyl chloride. After stirring at room temperature for 30 minutes, 20 ml of water were added and the reaction was extracted with 2 x 40 ml of methylene chloride. The combined organic layers were then dried ($Na_2SO_4$) and evaporated under reduced pressure to provide 1.2 g (94%) of product as an oil; $^1$H-NMR ($CDCl_3$) 7.73 (d, 2), 7.40 (d, 2H), 5.04 (m, 1H), 3.70 (m, 1H), 3.55 (dd, 1H), 3.05 (m, 1H), 3.0 (s, 3H), 2.83 (m, 1H), 2.62 (dd, 1H), 2.40 (s, 3H), 2.23 (s, 3H), 2.10 (m, 1H), 1.82 (m, 1H).

EXAMPLE 7

(2S,4R)-1-Methyl-2-[(methanesulfonylamino)methyl]-4-methanesulfonyloxypyrrolidine (VII, $R=R^2=R^3=CH_3$)

To title product of Example 4 (100 mg, 0.76 mmol) in 2 ml of tetrahydrofuran was added 0.21 ml (1.52 mmol) of triethylamine and 0.118 ml (1.52 mmol) of methanesulfonyl chloride and the mixture was allowed to stir at 0°C for one hour and at room temperature for an additional hour. Then 2 ml of water were added and the mixture was extracted with 2 x 2 ml of methylene chloride. The combined organic layers were dried ($MgSO_4$) and evaporated under reduced pressure to provide 140 mg (64%) of present title product as an oil; $^1$H-NMR ($CDC1_3$) 5.05 (m, 1H), 3.50 (dd, 1H), 3.17 (m, 1H), 3.0 (s, 3H), 2.95 (s, 3H), 2.80 (m, 1H), 2.58 (dd, 1H), 2.30 (s, 3H), 2.25-2.1 (m, 3H).
CMR($CDC1_3$) : 78.4, 62.3, 61.9, 42.5, 40.1, 39.8, 38.2, 35.4. MS: M+1 287(20), 191(17), 178(100).

EXAMPLE 8

(1S,4S)-2-Methyl-5-(4-methylbenzenesulfonyl)2,5-diazabicyclo[2.2.1]heptane (VIII, $R=CH_3$, $R^2=$ $pCH_3C_6H_4$)

To title product of Example 6 (760 mg, 5.52 mmol) was added 760 mg (5.52 mmol) of $K_2CO_3$. After stirring the mixture for 24 hours, the solvent was removed under reduced pressure and 20 ml of water were added. The aqueous layer was then extracted with 2 x 40 ml of methylene chloride and the combined organic layers were dried ($MgSO_4$) and evaporated under reduced pressure to provide 470 mg (64%) of present title product as a solid; mp 87-88°C; $^{13}$C-NMR ($CDCl_3$) 143.5, 135.4, 129.8, 127.4, 62.9, 61.1, 61.0, 49.9, 40.2, 34.9, 21.5.
Anal. C 58.73, H 6.90, N 10.51, S 12.26, calcd. C 58.62, H 6.81, N 10.52, S 12.04; $[alpha]_D = +18.69°$ (c=1.18, $CH_3OH$).

EXAMPLE 9

(1S,4S)-2-(Methanesulfonyl)-5-methyl-2,5diazabicyclo[2.2.1]heptane (VIII, $R=R^2=CH_3$)

By the method of the preceding Example, title product of Example 7 (110 mg, 0.38 mmol) was converted to present title product as an oil, purified by chromatography on silica gel; 44 mg (60%); $^1$H-NMR ($CDCl_3$) 4.27 (m, 1H), 3.55 (dd, 1H), 3.5 (s, 3H), 3.20 (dd, 1H), 2.84 (m, 3H), 1.92 (m, 1H), 1.71 (m, 1H); $^{13}$C-NMR ($CDCl_3$) : 63.1, 61.4, 60.6, 50.6, 40.8, 38.5, 35.7.

EXAMPLE 10

(1S,4S)-2-methyl-2,5-diazabicyclo[2.2.1]heptane (IX, $R=CH_3$)

Title product of Example 8 (60 g, 225 mmol) was suspended in 900 ml of 30% HBr in $CH_3COOH$, stirred for six hours at room temperature, then reduced to $\frac{1}{4}$ volume at the water aspirator, the residue diluted with 1800 ml of ethyl acetate, and the resulting precipitated solids recovered by filtration. These solids were recrystallized by dissolving in the minimum necessary $CH_3OH$ at reflux, cooling and the addition of 400 ml of isopropyl alcohol to yield 48 g (81%) of present, purified title product; mp 258-259°C; $^1$H-NMR ($D_2O$) 4.73 (m, 1H), 4.62 (m, 1H), 3.8-3.6 (m, 4H), 3.08 (s, 3H) 2.65 (m, 1H), 2.35 (m, 1H); $[alpha]_D = +13.21°$ (c=0.946, $CH_3OH$).

EXAMPLE 11

trans-4-Hydroxy-L-proline Methyl Ester Hydrochloride (X, $R^1=CH_3$)

Anhydrous HCl was bubbled through a stirred suspension of trans-4-hydroxy-L-proline (80 g, 0.61 mol) in 500 ml anhydrous methanol until the mixture was homogeneous. The reaction was heated to reflux for five hours, and the volume of the solvent then reduced by one half. Ether (100 ml) was added, and the mixture kept in a freezer overnight. The resulting precipitate was filtered, washed with ether and dried under reduced pressure to yield 111 g of present title product (93% yield). mp 170-172°C.

EXAMPLE 12

Methyl (2S,4R)-1-(4-Methylbenzenesulfonyl)-4-(4-methyl-benzenesulfonyloxy) pyrrolidine-2-carboxylate (XII, $R^1=CH_3$, $R^2=R^4=pCH_3C_6H_4$)

Title product of the preceding Example (15 g, 83.1 mmol) was stirred with 150 ml pyridine and 11.5 ml of triethylamine at 0°C for 30 minutes. p-Toluenesulfonyl chloride (34.8 g, 181.9 mmol) was added portionwise, maintaining a temperature of 0-5°C. The mixture was stirred 18 hours at 0°C, then added to two volumes of ice cold water, stirred at room temperature for one hour, and present title product recovered by filtration and dried in vacuo at 30°C for 48 hours to yield 38 g (99%) of present title product, mp 94-95°C.

EXAMPLE 13

(2S,4S)-$N^2$-Methyl-1-(4-methylbenzenesulfonyl)-4-(4-methylbenzenesulfonyloxy)pyrrolidine-2-carboxamide (XIII, $R=CH_3$, $R^2=R^4=pCH_3C_6H_4$)

Water (400 ml) was saturated with methylamine gas (20 minutes). Title product of the preceding Example (41 g, 89.4 mmol) was added and the resulting slurry stirred for 6 days. Present title product (25.2 g, 62%) was recovered as a white solid by filtration, mp 147-149°C; $[alpha]_D^{25}$ = -80.70 (c= 1.108, methanol); Anal. C 53.25, H 5.24, N 6.05, calcd. C 53.08, H 5.35, N 6.19.

EXAMPLE 14

(1S,4S)-2-Methyl-5-(4-methylbenzenesulfonyl)-2,5-diazabicyclo[2.2.1]heptane (VIII, $R=CH_3$, $R^2=pCH_3C_6H_4$)

To title product of the preceding Example (2.0 g, 4.42 mmol) in 20 ml tetrahydrofuran was added $LiAlH_4$ (750 mg, 19.9 mmol). The reaction mixture was stirred 24 hours, then quenched by the addition of 3 ml $H_2O$ and 0.75 ml 15% NaOH and extracted 2 x 15 ml $CH_2Cl_2$. The combined extracts were dried ($MgSO_4$) and stripped to yield 1 g (85%) of title product as a white solid identical with the product of Example 8.

**Claims**

**Claims for the following Contracting States : AT, BE, CH, DE, DK, FR, GB, GR, IT, LI, LU, NL, SE**

1. A process for the preparation of an optically active 2,5-diazabicyclo[2.2.1]heptane of formula

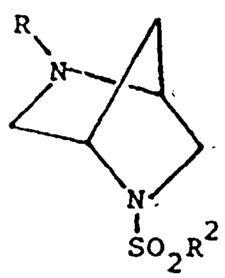

, ---(VIII)

wherein R is ($C_1$-$C_6$)alkyl; $R^2$ is ($C_1$-$C_6$)alkyl, trifluoromethyl or

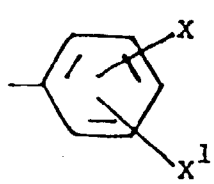

;

and X and $x^1$ are each independently hydrogen,
($C_1$-$C_6$)alkyl, bromo, chloro, trifluoromethyl, methoxy or nitro; which comprises the steps of:

(a) reductive alkylation of an optically active hydroxyproline of the relative or absolute stereochemical formula

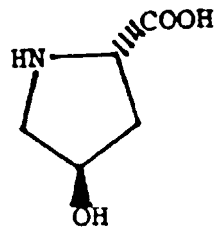

---(I)

to form an N-alkylhydroxyproline of the relative or absolute stereochemical formula

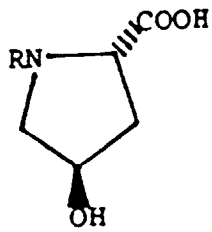

, ---(II)

(b) esterification of said N-alkylhydroxyproline with an alcohol of the formula

$R^1OH$,

wherein $R^1$ is ($C_1$-$C_6$)alkyl, in the presence of an acid catalyst to form an ester of the relative or absolute stereochemical formula

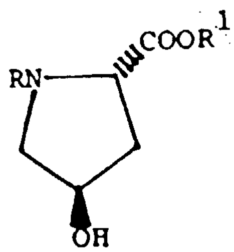

; ---(III)

(c) ammonolysis of said ester with $NH_3$ to form an amide of the relative or absolute stereochemical formula

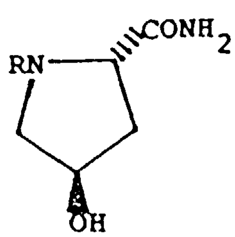

; ---(IV)

(d) hydride reduction of said amide to form an aminomethyl derivative of the relative or absolute stereochemical formula

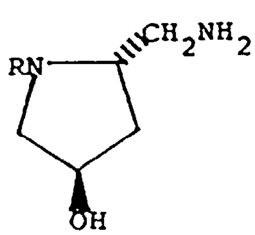

; ---(V)

(e) stepwise N-sulfonylation followed by O-sulfonylation of said aminomethyl derivative, each step being conducted in the presence of at least one equivalent of base, to form a sulfonamide of the relative or absolute stereochemical formula

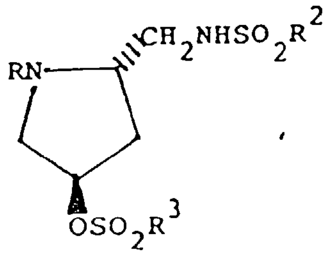 , ---(VII)

wherein $R^2$ is as defined above and $R^3$ is $(C_1-C_6)$ alkyl,or concurrent N- and O-sulfonylation, in the presence of at least two equivalents of base, to form a sulfonamide of the formula (VII) wherein $R^2$ is limited to $(C_1-C_6)$ alkyl and $R^2$ and $R^3$ each have the same value;

(f) contacting said sulfonamide with at least one molar equivalent of an alkali metal carbonate in a reaction inert solvent until the conversion of said sulfonamide to said compound of the formula (VIII) is substantially complete.

**2.** A process of claim 1 for a 2,5-diazabicyclo[2.2.1]heptane of the absolute stereochemical formula (VIII).

**3.** A process of claim 2 wherein R is methyl, $R^2$ is methyl or 4-methylphenyl and $R^3$ is methyl.

**4.** A process of claim 3 wherein the alkali metal carbonate is $K_2CO_3$ and the solvent is methanol.

**5.** A process for the preparation of an optically active 2,5-diazabicyclo[2.2.1]heptane of formula

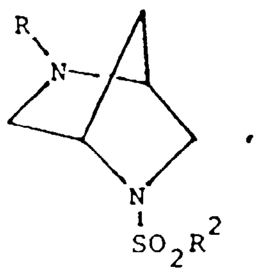 , ---(VIII)

wherein R is $(C_1-C_6)$alkyl; $R^2$ is $(C_1-C_6)$alkyl, trifluoromethyl or

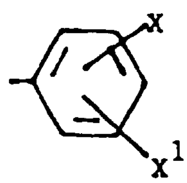 ;

and X and $x^1$ are each independently hydrogen, $(C_1-C_6)$alkyl, bromo, chloro, trifluoromethyl, methoxy or nitro; which comprises the steps of

(a) esterification of a hydroxyproline of the relative or absolute stereochemical formula

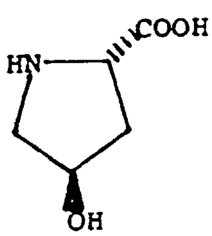 , ---(I)

with an alcohol of the formula

$R^1OH$,

wherein $R^1$ is $(C_1-C_6)$alkyl, to form a carboxylate ester of the relative or absolute stereochemical formula

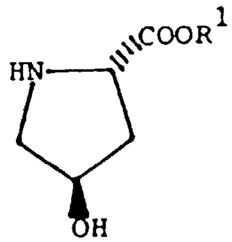 , --- (X)

wherein $R^1$ is $(C_1-C_6)$alkyl;

(b)stepwise N-sulfonylation followed by O-sulfonylation, each step being conducted in the presence of at least one equivalent of base, to form a sulfonate of the relative or absolute stereochemical formula

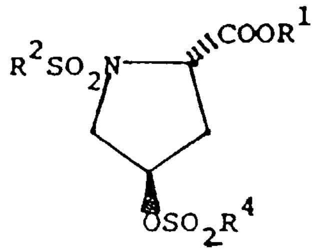 ; --- (XII)

wherein $R^4$ is independently a value of $R^2$ as defined above; or concurrent N- and O-sulfonylation, in the presence of at least two equivalents of base, to form a sulfonate of the formula (XII) wherein $R^2$ and $R^4$ have the same value;

(c) ammonolysis of said sulfonate with an amine of the formula

$$RNH_2$$

to form a carboxamide of the relative or absolute stereochemical formula

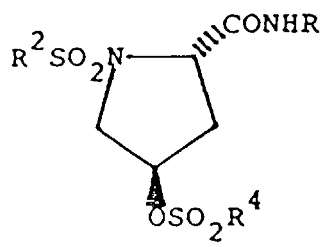 ; --- (XIII)

(d) hydride reduction of said carboxamide in a reaction inert solvent to form said compound of the formula (VIII).

**6.** A process of claim 5 for a 2,5-diazabicyclo-[2.2.1]heptane of the absolute stereochemical formula (VIII).

**7.** A process of claim 6 wherein R and $R^1$ are each methyl and $R^2$ and $R^4$ are the same and are each methyl or 4-methylphenyl.

**8.** A process of claim 7 wherein the hydride reducing agent is lithium aluminum hydride and the solvent is an ether.

**9.** A compound of the absolute stereochemical formula

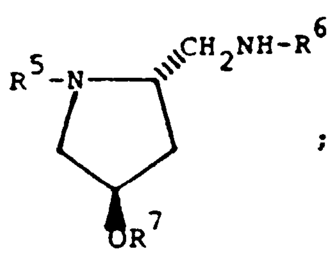 ; --- (XV)

wherein in a first alternative,

$R^5$ is ($C_1$-$C_6$)alkyl;
$R^6$ is $SO_2R^2$;
$R^2$ is ($C_1$-$C_6$)alkyl, trifluoromethyl or

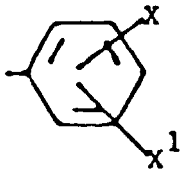 ;

X and $X^1$ are each independently hydrogen,

($C_1$-$C_6$)alkyl, bromo, chloro, trifluoromethyl, methoxy or nitro;

$R^7$ is hydrogen or $SO_2R^3$; and
$R^3$ is ($C_1$-$C_6$)alkyl; or
in a second alternative,
$R^6$ is ($C_1$-$C_6$)alkyl;
$R^5$ is $SO_2R^2$;
$R^2$ is as defined above;
$R^7$ is $SO_2R^4$; and
$R^4$ is independently a value of $R^2$ as defined above.

**10.** A compound of claim 9 in the first alternative wherein $R^5$ is methyl, $R^7$ is hydrogen or $SO_2CH_3$, $R^6$ is $SO_2R^2$ and $R^2$ is methyl or 4-methylphenyl.

**11.** A compound of claim 9 in the second alternative wherein $R^6$ is methyl, $R^5$ is $SO_2R^2$, and $R^2$ and $R^4$ are the same and are each methyl or 4-methylphenyl.

**Claims for the following Contracting State : ES**

**1.** A process for the preparation of an optically active 2,5-diazabicyclo[2.2.1]heptane of formula

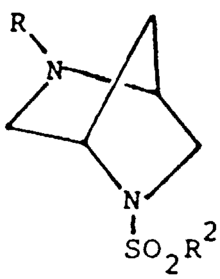 , ---(VIII)

wherein R is ($C_1$-$C_6$)alkyl; $R^2$ is ($C_1$-$C_6$)alkyl, trifluoromethyl or

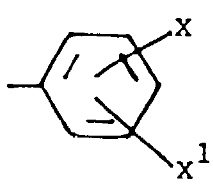 ;

and X and $X^1$ are each independently hydrogen,
($C_1$-$C_6$)alkyl, bromo, chloro, trifluoromethyl, methoxy or nitro; which comprises the steps of:

(a) reductive alkylation of an optically active hydroxyproline of the relative or absolute stereochemical formula

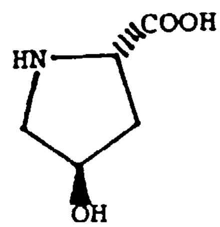

---(I)

to form an N-alkylhydroxyproline of the relative or absolute stereochemical formula

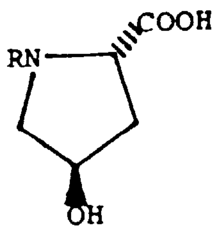

, ---(II)

(b) esterification of said N-alkylhydroxyproline with an alcohol of the formula

$R^1OH$,

wherein $R^1$ is $(C_1\text{-}C_6)$alkyl, in the presence of an acid catalyst to form an ester of the relative or absolute stereochemical formula

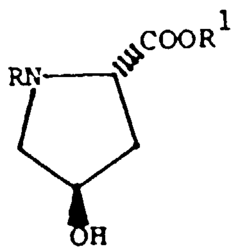

; ---(III)

(c) ammonolysis of said ester with $NH_3$ to form an amide of the relative or absolute stereochemical formula

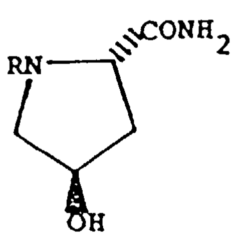

; ---(IV)

(d) hydride reduction of said amide to form an aminomethyl derivative of the relative or absolute stereochemical formula

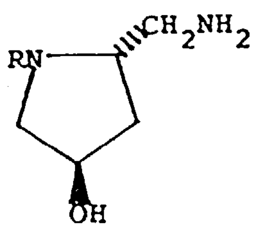

; ---(V)

(e) stepwise N-sulfonylation followed by O-sulfonylation of said aminomethyl derivative, each step being conducted in the presence of at least one equivalent of base, to form a sulfonamide of the relative or absolute stereochemical formula

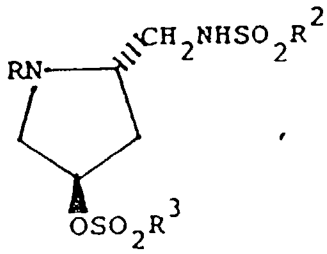

, ---(VII)

wherein $R^2$ is as defined above and $R^3$ is $(C_1\text{-}C_6)$ alkyl,or concurrent N- and O-sulfonylation, in the presence of at least two equivalents of base, to form a sulfonamide of the formula (VII) wherein $R^2$ is limited to $(C_1\text{-}C_6)$ alkyl and $R^2$ and $R^3$ each have the same value;

(f) contacting said sulfonamide with at least one molar equivalent of an alkali metal carbonate in a reaction inert solvent until the conversion of said sulfonamide to said compound of the formula (VIII) is substantially complete.

**2.** A process of claim 1 for a 2,5-diazabicyclo-[2.2.1]heptane of the absolute stereochemical formula (VIII).

**3.** A process of claim 2 wherein R is methyl, $R^2$ is methyl or 4-methylphenyl and $R^3$ is methyl.

**4.** A process of claim 3 wherein the alkali metal carbonate is $K_2CO_3$ and the solvent is methanol.

**5.** A process for the preparation of an optically active 2,5-diazabicyclo[2.2.1]heptane of formula

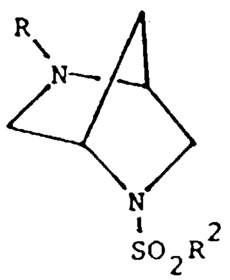

, ---(VIII)

wherein R is $(C_1\text{-}C_6)$alkyl; $R^2$ is $(C_1\text{-}C_6)$alkyl, trifluoromethyl or

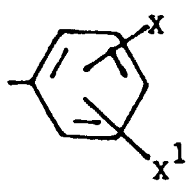

;

and X and $X^1$ are each independently hydrogen, $(C_1\text{-}C_6)$alkyl, bromo, chloro, trifluoromethyl, methoxy or nitro; which comprises the steps of

(a) esterification of a hydroxyproline of the relative or absolute stereochemical formula

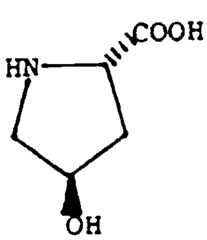

, ---(I)

with an alcohol of the formula

$R^1OH$,

wherein $R^1$ is $(C_1\text{-}C_6)$alkyl, to form a carboxylate ester of the relative or absolute stereochemical formula

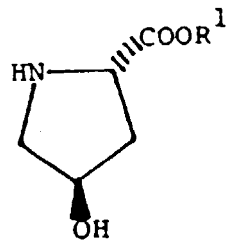

, ---(X)

wherein $R^1$ is $(C_1\text{-}C_6)$ alkyl;

(b)stepwise N-sulfonylation followed by O-sulfonylation, each step being conducted in the presence of at least one equivalent of base, to form a sulfonate of the relative or absolute stereochemical formula

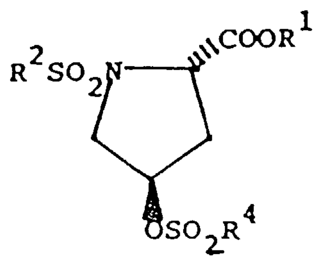

; ---(XII)

wherein $R^4$ is independently a value or $R^2$ as defined above; or concurrent N- and O-sulfonylation, in the presence of at least two equivalents of base, to form a sulfonate of the formula (XII) wherein $R^2$ and $R^4$ have the same value;

(c) ammonolysis of said sulfonate with an amine of the formula

$$RNH_2$$

to form a carboxamide of the relative or absolute stereochemical formula

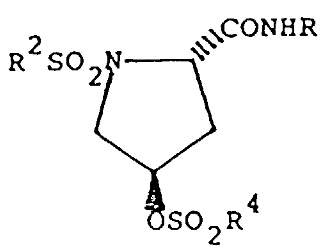

; ---(XIII)

(d) hydride reduction of said carboxamide in a reaction inert solvent to form said compound of the formula (VIII).

**6.** A process of claim 5 for a 2,5-diazabicyclo-[2.2.1]heptane of the absolute stereochemical formula (VIII).

**7.** A process of claim 6 wherein R and $R^1$ are each methyl and $R^2$ and $R^4$ are the same and are each methyl or 4-methylphenyl.

**8.** A process of claim 7 wherein the hydride reducing agent is lithium aluminum hydride and the solvent is an ether.

## Patentansprüche

### Patentansprüche für folgende Vertragsstaaten : AT, BE, CH, DE, DK, FR, GB, GR, IT, LI, LU, NL, SE

**1.** Verfahren zur Herstellung eines optisch aktiven 2,5-Diazabicyclo[2.2.1]heptans der Formel:

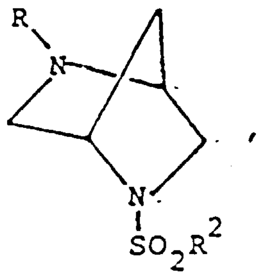

---(VIII)

worin R für $C_1$-$C_6$-Alkyl steht, $R^2$ $C_1$-$C_6$-Alkyl, Trifluormethyl oder

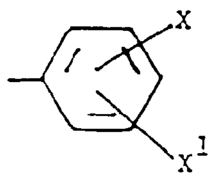

bedeutet und X und $X^1$ jeweils unabhängig voneinander Wasserstoff, $C_1$-$C_6$-Alkyl, Brom, Chlor, Trifluormethyl, Methoxy oder Nitro darstellen,
durch

(a) reduktive Alkylierung eines optisch aktiven Hydroxyprolins der relativen oder absoluten stereochemischen Formel:

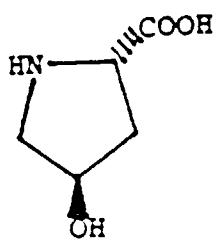

---(I)

unter Bildung eines N-Alkylhydroxyprolins der relativen oder absoluten stereochemischen Formel:

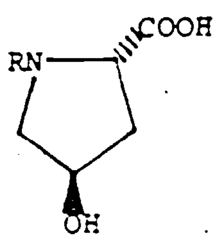

---(II)

(b) Verestern des obigen N-Alkylhydroxyprolins mit einem Alkohol der Formel

$R^1OH$,

worin $R^1$ für $C_1$-$C_6$-Alkyl steht, in Gegenwart eines sauren Katalysators unter Bildung eines Esters der relativen oder absoluten stereochemischen Formel:

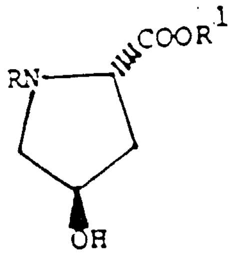

; ---(III)

(c) Ammonolyse des obigen Esters mit $NH_3$ unter Bildung eines Amids der relativen oder absoluten stereochemischen Formel:

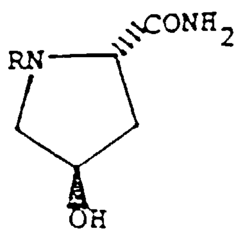

; ---(IV)

(d) Hydridreduktion des obigen Amids unter Bildung eines Aminomethylderivats der relativen oder absoluten stereochemischen Formel:

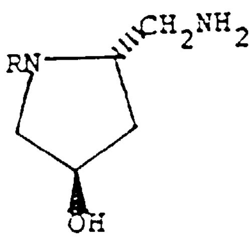

; ---(V)

(e) stufenweise N-Sulfonierung und anschließende O-Sulfonierung des obigen Aminomethylderivats, wobei jede Stufe in Anwesenheit mindestens eines Äquivalents Base durchgeführt wird, unter Bildung eines Sulfonamids der relativen oder absoluten stereochemischen Formel:

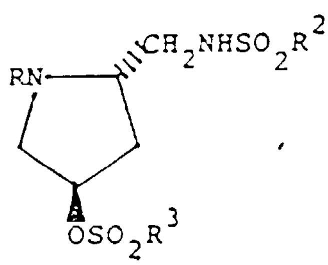

, ---(VII)

worin $R^2$ die oben angegebene Bedeutung besitzt und $R^3$ für $C_1$-$C_6$-Alkyl steht, oder gleichzeitige N- und O-Sulfonierung in Gegenwart von mindestens zwei Äquivalenten Base unter Bildung eines Sulfonamids der Formel (VII), wobei $R^2$ auf $C_1$-$C_6$-Alkyl beschränkt ist und $R^2$ und $R^3$ jeweils dieselbe Bedeutung besitzen, und

(f) Inberührungbringen des obigen Sulfonamids mit mindestens 1 Moläquivalent eines Alkalimetallcarbonats in einem reaktionsinerten Lösungsmittel, bis die Umwandlung des Sulfonamids in die Verbindung der Formel (VIII) im wesentlichen vollständig abgelaufen ist.

**2.** Verfahren nach Anspruch 1 zur Herstellung eines 2,5Diazabicyclo[2.2.1]heptans der absoluten stereochemischen Formel (VIII).

**3.** Verfahren nach Anspruch 2, wobei R für Methyl steht, $R^2$ Methyl oder 4-Methylphenyl bedeutet und $R^3$ Methyl ist.

**4.** Verfahren nach Anspruch 3, wobei das Alkalimetallcarbonat $K_2CO_3$ und das Lösungsmittel Methanol sind.

**5.** Verfahren zur Herstellung eines optisch aktiven 2,5-Diazabicyclo[2.2.1]heptans der Formel:

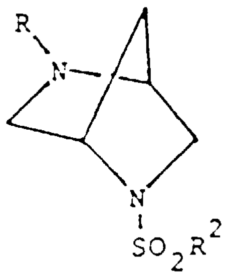

, ---(VIII)

worin R für $C_1$-$C_6$-Alkyl steht, $R^2$ $C_1$-$C_6$-Alkyl, Trifluormethyl oder

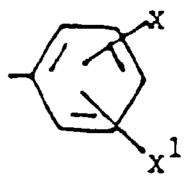

bedeutet und
X und $X^1$ jeweils Wasserstoff, $C_1$-$C_6$-Alkyl, Brom, Chlor, Trifluormethyl, Methoxy oder Nitro darstellen, durch

(a) Verestern eines Hydroxyprolins der relativen oder absoluten stereochemischen Formel:

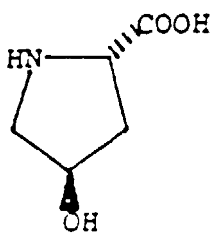

, ---(I)

mit einem Alkohol der Formel $R^1OH$,
worin $R^1$ für $C_1$-$C_6$-Alkyl steht, unter Bildung eines Carboxylatesters der relativen oder absoluten stereochemischen Formel:

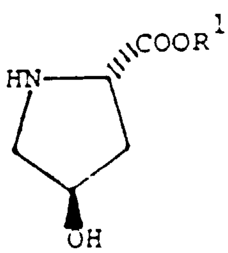

, ---(X)

worin $R^1$ für $C_1$-$C_6$-Alkyl steht,

(b) stufenweise N-Sulfonierung und anschließende O-Sulfonierung, wobei jede Stufe in Gegenwart mindestens eines Äquivalents Base durchgeführt wird, unter Bildung eines Sulfonats der folgenden relativen oder absoluten stereochemischen Formel:

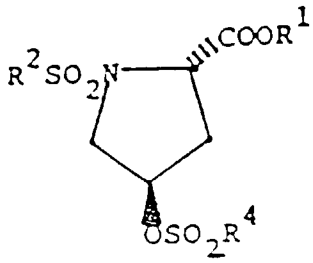

; --- (XII)

worin $R^4$ unabhängig irgendeine der obigen Bedeutungen von $R^2$ besitzen kann, oder gleichzeitige N- und O-Sulfonierung in Gegenwart von mindestens zwei Äquivalenten Base unter Bildung eines Sulfonats der Formel (XII), worin $R^2$ und $R^4$ dieselbe Bedeutung besitzen,

(c) Ammonolyse des Sulfonats mit einem Amin der Formel $RNH_2$ unter Bildung eines Carboxamids der relativen oder absoluten stereochemischen Formel:

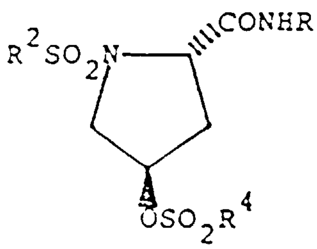

; --- (XIII)

und

(d) Hydridreduktion des Carboxamids in einem reaktionsinerten Lösungsmittel unter Bildung der Verbindung der Formel (VIII).

**6.** Verfahren nach Anspruch 5 zur Herstellung eines 2,5-Diazabicyclo[2.2.1]heptans der absoluten stereochemischen Formel (VIII).

**7.** Verfahren nach Anspruch 6, wobei R und $R^1$ jeweils Methyl bedeuten und $R^2$ und $R^4$ dieselbe Bedeutung besitzen und jeweils Methyl oder 4-Methylphenyl sind.

**8.** Verfahren nach Anspruch 7, wobei das Hydridreduktionsmittel Lithiumaluminiumhydrid und das Lösungsmittel ein Ether sind.

**9.** Verbindung der absoluten stereochemischen Formel:

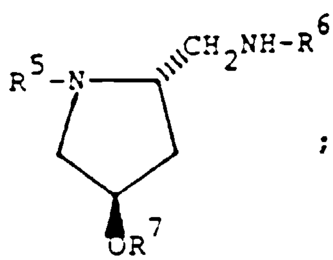

; --- (XV)

worin in einer ersten Alternative $R^5$ $C_1$-$C_6$-Alkyl bedeutet, $R^6$ für $SO_2R^2$ steht, $R^2$ $C_1$-$C_6$-Alkyl, Trifluormethyl oder

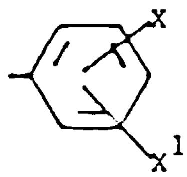

darstellt,
X und $X^1$ jeweils unabhängig voneinander für Wasserstoff, $C_1$-$C_6$-Alkyl, Brom, Chlor, Trifluormethyl, Methoxy oder Nitro stehen, $R^7$ Wasserstoff oder $SO_2R^3$ entspricht und $R^3$ $C_1$-$C_6$-Alkyl ist oder
in einer zweiten Alternative $R^6$ $C_1$-$C_6$-Alkyl bedeutet, $R^5$ $SO_2R^2$ darstellt, $R^2$ die obige Bedeutung besitzt, $R^7$ für $SO_2R^4$ steht und $R^4$ unabhängig voneinander eine der obigen Bedeutungen von $R^2$ besitzt.

**10.** Verbindung nach Anspruch 9 in der ersten Alternative, wobei $R^5$ für Methyl steht, $R^7$ Wasserstoff oder $SO_2CH_3$ darstellt, $R^6$ $SO_2R^2$ ist und $R^2$ Methyl oder 4-Methylphenyl darstellt.

**11.** Verbindung nach Anspruch 9 in der zweiten Alternative, wobei $R^6$ Methyl ist, $R^5$ $SO_2R^2$ bedeutet und $R^2$ und $R^4$ die gleiche Bedeutung besitzen und jeweils Methyl oder 4-Methylphenyl darstellen.

**Patentansprüche für folgende Vertragsstaat : ES**

**1.** Verfahren zur Herstellung eines optisch aktiven 2,5-Diazabicyclo[2.2.1]heptans der Formel:

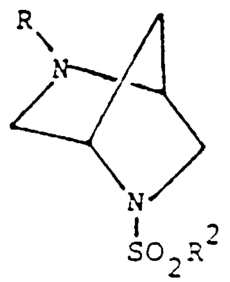

,   ---(VIII)

worin R für $C_1$-$C_6$-Alkyl steht, $R^2$ $C_1$-$C_6$-Alkyl, Trifluormethyl oder

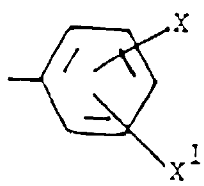

bedeutet und
X und $X^1$ jeweils unabhängig voneinander Wasserstoff, $C_1$-$C_6$-Alkyl, Brom, Chlor, Trifluormethyl, Methoxy oder Nitro darstellen,
durch

(a) reduktive Alkylierung eines optisch aktiven Hydroxyprolins der relativen oder absoluten stereochemischen Formel:

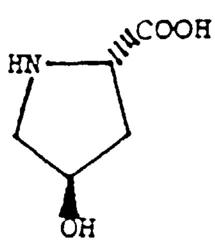

---(I)

unter Bildung eines N-Alkylhydroxyprolins der relativen oder absoluten stereochemischen Formel:

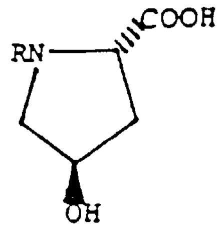

---(II)

(b) Verestern des obigen N-Alkylhydroxyprolins mit einem Alkohol der Formel

$R^1OH$,

worin $R^1$ für $C_1$-$C_6$-Alkyl steht, in Gegenwart eines sauren Katalysators unter Bildung eines Esters der relativen oder absoluten stereochemischen Formel:

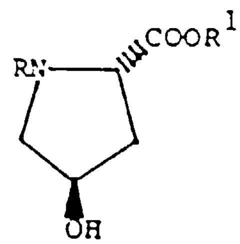

; ---(III)

(c) Ammonolyse des obigen Esters mit $NH_3$ unter Bildung eines Amids der relativen oder absoluten stereochemischen Formel:

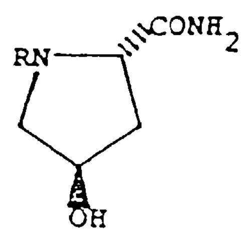

; ---(IV)

(d) Hydridreduktion des obigen Amids unter Bildung eines Aminomethylderivats der relativen oder absoluten stereochemischen Formel:

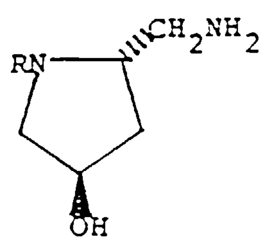

; ---(V)

(e) stufenweise N-Sulfonierung und anschließende O-Sulfonierung des obigen Aminomethylderivats, wobei jede Stufe in Anwesenheit mindestens eines Äquivalents Base durchgeführt wird, unter Bildung eines Sulfonamids der relativen oder absoluten stereochemischen Formel:

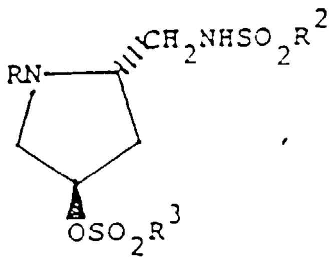

, ---(VII)

worin $R^2$ die oben angegebene Bedeutung besitzt und $R^3$ für $C_1$-$C_6$-Alkyl steht, oder gleichzeitige N- und O-Sulfonierung in Gegenwart von mindestens zwei Äquivalenten Base unter Bildung eines Sulfonamids der Formel (VII), wobei $R^2$ auf $C_1$-$C_6$-Alkyl beschränkt ist und $R^2$ und $R^3$ jeweils dieselbe Bedeutung besitzen, und

(f) Inberührungbringen des obigen Sulfonamids mit mindestens 1 Moläquivalent eines Alkalimetallcarbonats in einem reaktionsinerten Lösungsmittel, bis die Umwandlung des Sulfonamids in die Verbindung der Formel (VIII) im wesentlichen vollständig abgelaufen ist.

**2.** Verfahren nach Anspruch 1 zur Herstellung eines 2,5-Diazabicyclo[2.2.1]heptans der absoluten stereochemischen Formel (VIII).

**3.** Verfahren nach Anspruch 2, wobei R für Methyl steht, $R^2$ Methyl oder 4-Methylphenyl bedeutet und $R^3$ Methyl ist.

**4.** Verfahren nach Anspruch 3, wobei das Alkalimetallcarbonat $K_2CO_3$ und das Lösungsmittel Methanol sind.

**5.** Verfahren zur Herstellung eines optisch aktiven 2,5-Diazabicyclo[2.2.1]heptans der Formel:

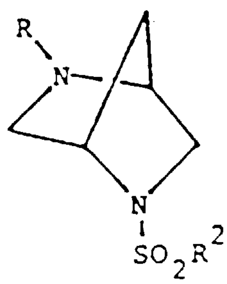

, ---(VIII)

worin R für $C_1$-$C_6$-Alkyl steht, $R^2$ $C_1$-$C_6$-Alkyl, Trifluormethyl oder

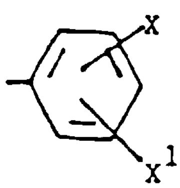

bedeutet und
X und $X^1$ jeweils Wasserstoff, $C_1$-$C_6$-Alkyl, Brom, Chlor, Trifluormethyl, Methoxy oder Nitro darstellen, durch

(a) Verestern eines Hydroxyprolins der relativen oder absoluten stereochemischen Formel:

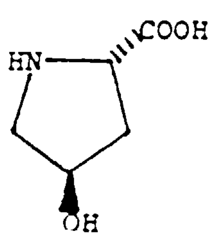

, ---(I)

mit einem Alkohol der Formel $R^1OH$,
worin $R^1$ für $C_1$-$C_6$-Alkyl steht, unter Bildung eines Carboxylatesters der relativen oder absoluten stereoche-

mischen Formel:

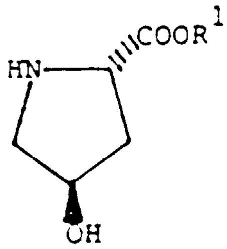 , ---(X)

worin $R^1$ für $C_1$-$C_6$-Alkyl steht,

(b) stufenweise N-Sulfonierung und anschließende O-Sulfonierung, wobei jede Stufe in Gegenwart mindestens eines Äquivalents Base durchgeführt wird unter Bildung eines Sulfonats der folgenden relativen oder absoluten stereochemischen Formel:

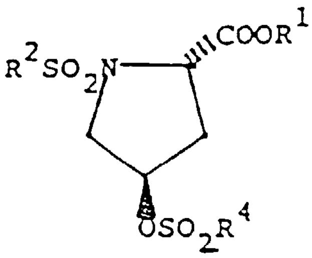 ; ---(XII)

worin $R^4$ unabhängig irgendeine der obigen Bedeutungen von $R^2$ besitzen kann, oder gleichzeitige N- und O-Sulfonierung in Gegenwart von mindestens zwei Äquivalenten Base unter Bildung eines Sulfonats der Formel (XII), worin $R^2$ und $R^4$ dieselbe Bedeutung besitzen,

(c) Ammonolyse des Sulfonats mit einem Amin der Formel $RNH_2$ unter Bildung eines Carboxamids der relativen oder absoluten stereochemischen Formel:

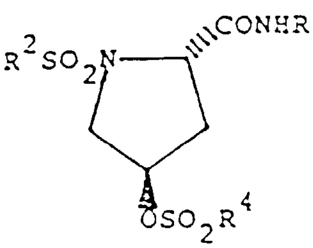 ; ---(XIII)

und

(d) Hydridreduktion des Carboxamids in einem reaktionsinerten Lösungsmittel unter Bildung der Verbindung der Formel (VIII).

**6.** Verfahren nach Anspruch 5 zur Herstellung eines 2,5-Diazabicyclo[2.2.1]heptans der absoluten stereochemischen Formel (VIII).

**7.** Verfahren nach Anspruch 6, wobei R und $R^1$ jeweils Methyl bedeuten und $R^2$ und $R^4$ dieselbe Bedeutung besitzen und jeweils Methyl oder 4-Methylphenyl sind.

**8.** Verfahren nach Anspruch 7, wobei das Hydridreduktionsmittel Lithiumaluminiumhydrid und das Lösungsmittel ein Ether sind.

## Revendications

**Revendications pour les Etats contractants suivants : AT, BE, CH, DE, DK, FR, GB, GR, IT, LI, LU, NL, SE**

1. Procédé de préparation d'un 2,5-diazabicyclo[2.2.1]heptane optiquement actif, de formule

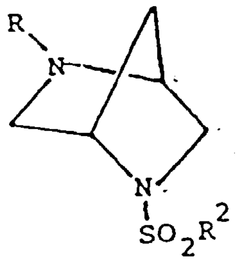

---(VIII)

dans laquelle R représente un groupe alkyle en $C_1$ à $C_6$ ; $R^2$ représente un groupe alkyle en $C_1$ à $C_6$, trifluorométhyle ou

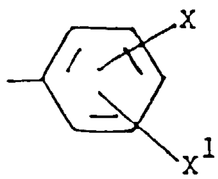

;

et X et $X^1$ représentent chacun indépendamment l'hydrogène, un groupe alkyle en $C_1$ à $C_6$, bromo, chloro, trifluorométhyle, méthoxy ou nitro ; qui comprend les étapes suivantes :

(a) alkylation réductrice d'une hydroxyproline optiquement active répondant à la formule stéréochimique relative ou absolue

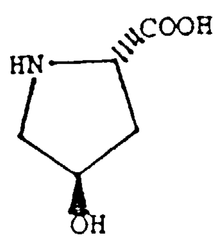

---(I)

pour former une N-alkylhydroxyproline répondant à la formule stéréochimique relative ou absolue

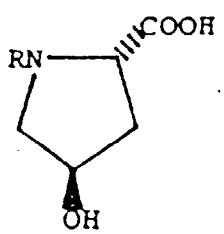

---(II)

(b) estérification de ladite N-alkylhydroxyproline avec un alcool de formule

$R^1OH$,

dans laquelle $R^1$ représente un groupe alkyle en $C_1$ à $C_6$, en présence d'un catalyseur acide pour former un ester répondant à la formule stéréochimique relative ou absolue

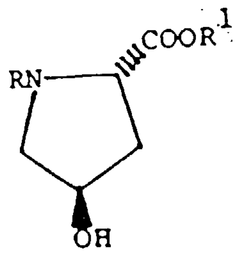

; ---(III)

(c) ammonolyse dudit ester avec $NH_3$ pour former un amide répondant à la formule stéréochimique relative ou absolue

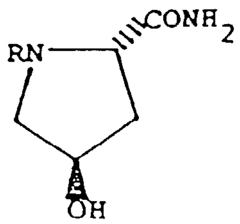

; ---(IV)

(d) réduction avec un hydrure de cet amide pour former un dérivé à fonction aminométhyle, répondant à la formule stéréochimique relative ou absolue

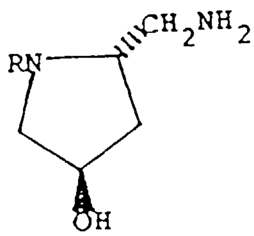

; ---(V)

(e) N-sulfonylation par étapes, suivie par une O-sulfonylation dudit dérivé à fonction aminométhyle, chaque étape étant conduite en présence d'au moins un équivalent d'une base, pour former un sulfonamide répondant à la formule stéréochimique relative ou absolue

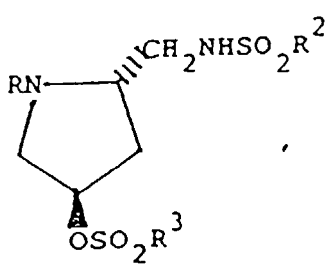

, ---(VII)

dans laquelle $R^2$ répond à la définition précitée et $R^3$ représente un groupe alkyle en $C_1$ à $C_6$, ou une N-sulfonylation et une O-sulfonylation conjointes, en présence d'au moins deux équivalents d'une base, pour former un sulfonamide de formule (VII) dans laquelle $R^2$ est limité à un groupe alkyle en $C_1$ à $C_6$ et $R^2$ et $R^3$ ont chacun la même valeur :

(f) mise en contact dudit sulfonamide avec au moins un équivalent molaire d'un carbonate de métal alcalin dans un solvant inerte vis-à-vis de la réaction jusqu'à ce que la transformation dudit sulfonamide en ledit composé de formule (VIII) soit pratiquement total.

**2.** Procédé suivant la revendication 1, pour un 2,5-diazabicyclo[2.2.1]heptane répondant à la formule stéréochimique absolue (VIII).

**3.** Procédé suivant la revendication 2, dans lequel R représente un groupe méthyle, $R^2$ représente un groupe méthyle ou 4-méthyphényle et $R^3$ représente un groupe méthyle.

**4.** Procédé suivant la revendication 3, dans lequel le carbonate de métal alcalin consiste en $K_2CO_3$ et le solvant consiste en méthanol.

**5.** Procédé de préparation d'un 2,5-diazabicyclo[2.2.1]heptane optiquement actif répondant à la formule

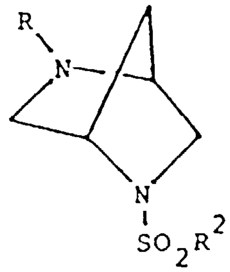

, ---(VIII)

dans laquelle R représente un groupe alkyle en $C_1$ à $C_6$ ; $R^2$ représente un groupe alkyle en $C_1$ à $C_6$, trifluorométhyle ou

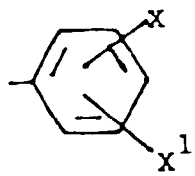

;

et X et $X^1$ représentent chacun indépendamment l'hydrogène, un groupe alkyle en $C_1$ à $C_6$, bromo, chloro, trifluorométhyle, méthoxy ou nitro, qui comprend les étapes suivantes :

(a) estérification d'une hydroxyproline répondant à la formule stéréochimique relative ou absolue

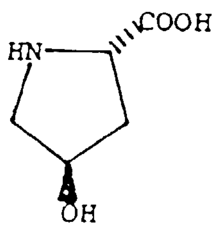

, ---(I)

avec un alcool de formule

$R^1OH$,

dans laquelle $R^1$ représente un groupe alkyle en $C_1$ à $C_6$, pour former un ester carboxylique répondant à la formule stéréochimique relative ou absolue

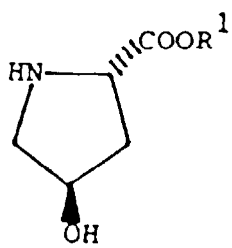

, ---(X)

dans laquelle $R^1$ représente un groupe alkyle en $C_1$ à $C_6$ ;

(b) N-sulfonylation par étapes, suivie par une O-sulfonylation, chaque étape étant conduite en présence d'au moins un équivalent d'une base, pour former un sulfonate répondant à la formule stéréochimique relative ou absolue

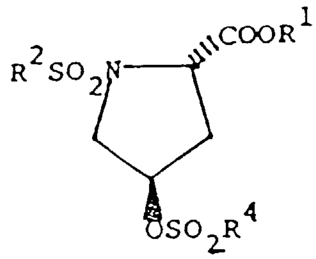
; ---(XII)

dans laquelle $R^4$ représente indépendamment une valeur de $R^2$ répondant à la définition précitée ; ou une N-sulfonylation et une O-sulfonylation conjointes, en présence d'au moins deux équivalents d'une base, pour former un sulfonate de formule (XII) dans laquelle $R^2$ et $R^4$ ont la même valeur ;

(c) ammonolyse dudit sulfonate avec une amine de formule

$$RNH_2$$

pour former un carboxamide répondant à la formule stéréochimique relative ou absolue

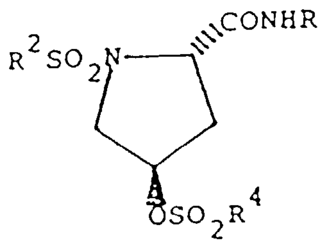
; ---(XIII)

(d) réduction au moyen d'un hydrure dudit carboxamide dans un solvant inerte vis-à-vis de la réaction pour former ledit composé de formule (VIII).

**6.** Procédé suivant la revendication 5 pour un 2,5-diazabicyclo[2.2.1]heptane répondant à la formule stéréochimique absolue (VIII).

**7.** Procédé suivant la revendication 6, dans lequel R et $R^1$ représentent chacun un groupe méthyle et $R^2$ et $R^4$ sont identiques et représentent chacun un groupe méthyle ou 4-méthylphényle.

**8.** Procédé suivant la revendication 7, dans lequel l'agent réducteur consistant en un hydrure est l'hydrure de lithium aluminium et le solvant est un éther.

**9.** Composé répondant à la formule stéréochimique absolue

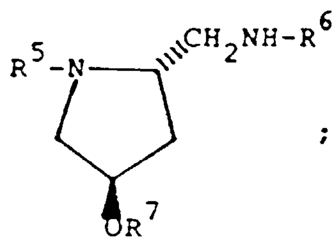
; ---(XV)

dans laquelle, dans une première variante,

$R^5$ représente un groupe alkyle en $C_1$ à $C_6$,
$R^6$ représente un groupe $SO_2R^2$ ;
$R^2$ représente un groupe alkyle en $C_1$ à $C_6$, trifluorométhyle ou

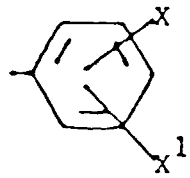

;

X et $X_1$ représentent chacun indépendamment l'hydrogène, un groupe alkyle en $C_1$ à $C_6$, bromo, chloro, trifluorométhyle, méthoxy ou nitro ;
$R^7$ représente l'hydrogène ou un groupe $SO_2R^3$ ; et
$R^3$ représente un groupe alkyle en $C_1$ à $C_6$ ; ou,

dans une seconde variante,

$R^6$ représente un groupe alkyle en $C_1$ à $C_6$,
$R^5$ représente un groupe $SO_2R^2$ ;
$R^2$ répond à la définition précitée ;
$R^7$ représente un groupe $SO_2R^4$ ; et
$R^4$ représente indépendamment une valeur de $R^2$ répondant à la définition précitée.

**10.** Composé suivant la revendication 9, dans la première variante, dans lequel $R^5$ représente un groupe méthyle, $R^7$ représente l'hydrogène ou un groupe $SO_2CH_3$, $R^6$ représente un groupe $SO_2R^2$ et $R^2$ représente un groupe méthyle ou 4-méthylphényle.

**11.** Composé suivant la revendication 9, dans la seconde variante, dans lequel $R^6$ représente un groupe méthyle, $R^5$ représente un groupe $SO_2R^2$ et $R^2$ et $R^4$ sont identiques et représentent chacun un groupe méthyle ou 4-méthylphényle.

**Revendications pour l'Etat contractant suivant : ES**

**1.** Procédé de préparation d'un 2,5-diazabicyclo[2.2.1]heptane optiquement actif, de formule

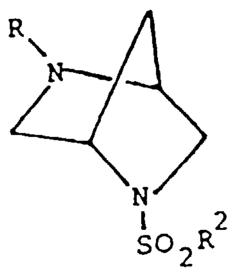

, ---(VIII)

dans laquelle R représente un groupe alkyle en $C_1$ à $C_6$ ; $R^2$ représente un groupe alkyle en $C_1$ à $C_6$, trifluorométhyle ou

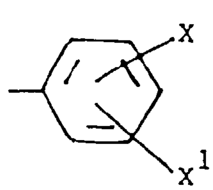

;

et X et $X^1$ représentent chacun indépendamment l'hydrogène, un groupe alkyle en $C_1$ à $C_6$, bromo, chloro, trifluorométhyle, méthoxy ou nitro ; qui comprend les étapes suivantes :

(a) alkylation réductrice d'une hydroxyproline optiquement active répondant à la formule stéréochimique relative ou absolue

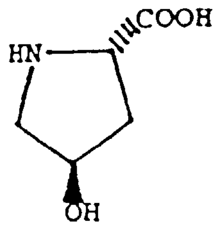

---(I)

pour former une N-alkylhydroxyproline répondant à la formule stéréochimique relative ou absolue

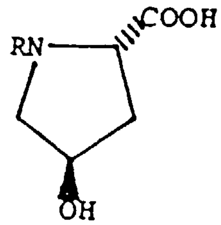

; ---(II)

(b) estérification de ladite N-alkylhydroxyproline avec un alcool de formule

$R^1OH$,

dans laquelle $R^1$ représente un groupe alkyle en $C_1$ à $C_6$, en présence d'un catalyseur acide pour former un ester répondant à la formule stéréochimique relative ou absolue

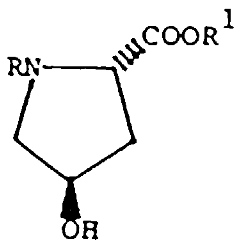

; ---(III)

(c) ammonolyse dudit ester avec $NH_3$ pour former un amide répondant à la formule stéréochimique relative ou absolue

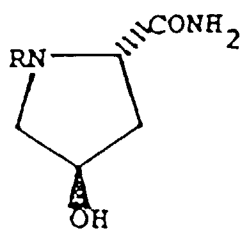

; ---(IV)

(d) réduction avec un hydrure de cet amide pour former un dérivé à fonction aminométhyle, répondant à la formule stéréochimique relative ou absolue

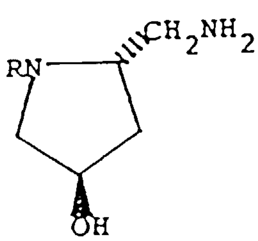

; ---(V)

(e) N-sulfonylation par étapes, suivie par une O-sulfonylation dudit dérivé à fonction aminométhyle, chaque étape étant conduite en présence d'au moins un équivalent d'une base, pour former un sulfonamide répondant

à la formule stéréochimique relative ou absolue

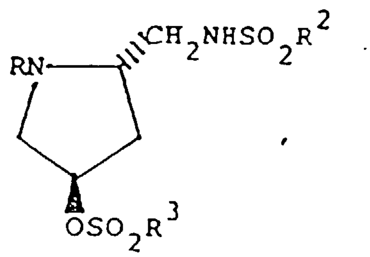

---(VII)

dans laquelle $R^2$ répond à la définition précitée et $R^3$ représente un groupe alkyle en $C_1$ à $C_6$, ou une N-sulfonylation et une O-sulfonylation conjointes, en présence d'au moins deux équivalents d'une base, pour former un sulfonamide de formule (VII) dans laquelle $R^2$ est limité à un groupe alkyle en $C_1$ à $C_6$ et $R^2$ et $R^3$ ont chacun la même valeur :

(f) mise en contact dudit sulfonamide avec au moins un équivalent molaire d'un carbonate de métal alcalin dans un solvant inerte vis-à-vis de la réaction jusqu'à ce que la transformation dudit sulfonamide en ledit composé de formule (VIII) soit pratiquement total.

**2.** Procédé suivant la revendication 1, pour un 2,5-diazabicyclo[2.2.1]heptane répondant à la formule stéréochimique absolue (VIII).

**3.** Procédé suivant la revendication 2, dans lequel R représente un groupe méthyle, $R^2$ représente un groupe méthyle ou 4-méthyphényle et $R^3$ représente un groupe méthyle.

**4.** Procédé suivant la revendication 3, dans lequel le carbonate de métal alcalin consiste en $K_2CO_3$ et le solvant consiste en méthanol.

**5.** Procédé de préparation d'un 2,5-diazabicyclo[2.2,1]heptane optiquement actif de formule

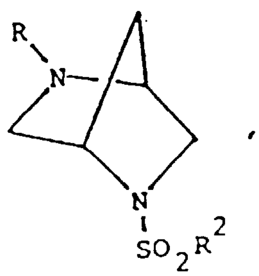

---(VIII)

dans laquelle R représente un groupe alkyle en $C_1$ à $C_6$ ; $R^2$ représente un groupe alkyle en $C_1$ à $C_6$, trifluorométhyle ou

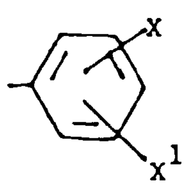

;

et X et $X^1$ représentent chacun indépendamment l'hydrogène, un groupe alkyle en $C_1$ à $C_6$, bromo, chloro, trifluorométhyle, méthoxy ou nitro, qui comprend les étapes suivantes :

(a) estérification d'une hydroxyproline répondant à la formule stéréochimique relative ou absolue

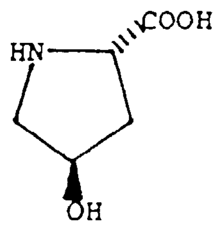 , ---(I)

avec un alcool de formule

$R^1OH$,

dans laquelle $R^1$ représente un groupe alkyle en $C_1$ à $C_6$, pour former un ester carboxylique répondant à la formule stéréochimique relative ou absolue

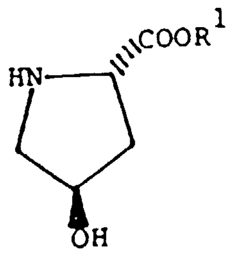 , ---(X)

dans laquelle $R^1$ représente un groupe alkyle en $C_1$ à $C_6$ ;
(b) N-sulfonylation par étapes, suivie par une O-sulfonylation, chaque étape étant conduite en présence d'au moins un équivalent d'une base, pour former un sulfonate répondant à la formule stéréochimique relative ou absolue

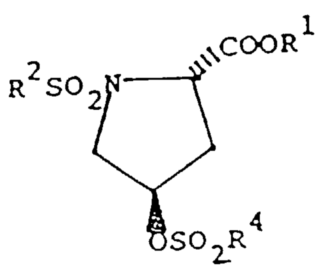 ; ---(XII)

dans laquelle $R^4$ représente indépendamment une valeur de $R^2$ répondant à la définition précitée ; ou une N-sulfonylation et une O-sulfonylation conjointes, en présence d'au moins deux équivalents d'une base, pour former un sulfonate de formule (XII) dans laquelle $R^2$ et $R^4$ ont la même valeur ;
(c) ammonolyse dudit sulfonate avec une amine de formule

$RNH_2$

pour former un carboxamide répondant à la formule stéréochimique relative ou absolue

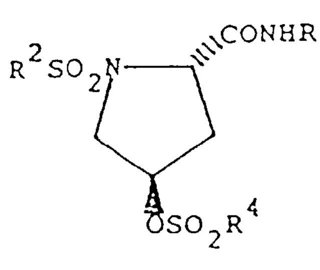 ; ---(XIII)

(d) réduction au moyen d'un hydrure dudit carboxamide dans un solvant inerte vis-à-vis de la réaction pour former ledit composé de formule (VIII).

**6.** Procédé suivant la revendication 5 pour un 2,5-diazabicyclo[2.2.1]heptane répondant à la formule stéréochimique absolue (VIII).

**7.** Procédé suivant la revendication 6, dans lequel R et $R^1$ représentent chacun un groupe méthyle et $R^2$ et $R^4$ sont identiques et représentent chacun un groupe méthyle ou 4-méthylphényle.

**8.** Procédé suivant la revendication 7, dans lequel l'agent réducteur consistant en un hydrure est l'hydrure de lithium-aluminium et le solvant consiste en un éther.